## Europäisches Patentamt
## European Patent Office
## Office européen des brevets

(11) Publication number: **0 145 234**
**B1**

(12) # EUROPEAN PATENT SPECIFICATION

(45) Date of publication of patent specification: **21.12.88**

(51) Int. Cl.⁴: **C 10 G 3/00**

(21) Application number: **84307546.6**

(22) Date of filing: **01.11.84**

(54) **Multistage process and apparatus for converting oxygenates into hydrocarbons.**

(30) Priority: **03.11.83 US 548377**

(43) Date of publication of application:
**19.06.85 Bulletin 85/25**

(45) Publication of the grant of the patent:
**21.12.88 Bulletin 88/51**

(84) Designated Contracting States:
**BE DE FR GB IT NL**

(56) References cited:
**US-A-3 928 483**
**US-A-4 413 153**

(73) Proprietor: **MOBIL OIL CORPORATION**
**150 East 42nd Street**
**New York New York 10017 (US)**

(72) Inventor: **Tabak, Samuel Allen**
**204 East Pine Street**
**Wenonah New Jersey 08090 (US)**

(74) Representative: **Cooper, John Anthony**
**Mobil Court 3 Clements Inn**
**London WC2A 2EB (GB)**

EP 0 145 234 B1

## Description

This invention relates to a multistage process and apparatus for converting oxygenates, such as methanol or dimethyl ether (DME), into liquid hydrocarbons. More especially, it provides a continuous process for producing distillate-range fuel products by catalytically dehydrating an oxygenate feedstock to produce a lower olefinic intermediate, oligomerizing the olefins to produce distillate/gasoline, and hydrogenating the distillate product to yield a stabilized product useful as a diesel or other fuel.

Recent developments in both zeolite catalysts and hydrocarbon conversion processes have created interest in utilizing olefinic feedstocks for producing $C_5^+$ gasoline and diesel fuel. In addition to the basic work derived from ZSM-5 type zeolite catalysts, a number of discoveries have contributed to the development of a new industrial process, known as the Mobil Olefins to Gasoline/Distillate (MOGD) process. This process has commercial significance as a safe and environmentally acceptable technique for utilizing feedstocks that contain lower olefins, especially $C_2$—$C_5$ alkenes and may supplant conventional alkylation units. U.S. Patents 3,960,978 and 4,021,502 describe the conversion of $C_2$—$C_5$ olefins, alone or in admixture with paraffinic components, into higher hydrocarbons over crystalline zeolites having controlled acidity. Improved processing techniques applicable to the MOGD system are described in U.S. Patents 4,150,062, 4,211,640 and 4,227,992.

The conversion of lower olefins, especially propene and butenes, over HZSM-5 is effective at moderately elevated temperatures and pressures. The conversion products are valuable liquid fuels, especially the $C_5^+$ aliphatic and aromatic hydrocarbons. Olefinic gasoline is produced in good yield by the MOGD process and may be recovered as a product or recycled to the reactor system for further conversion into distillate-range products. Operating details for typical MOGD units are described in U.S. Patents 4,433,185 and 4,456,779.

In addition to their use as shape selective oligomerization catalysts, the medium pores ZSM-5 type zeolites are useful for converting methanol and other lower aliphatic alcohols or corresponding ethers into olefins. Particular interest has been directed to a catalytic process for converting low cost methanol into valuable hydrocarbons rich in ethene and $C_3^+$ alkenes. Various processes are described in U.S. Patents 3,894,107 3,928,483 and 4,025,571 and in European Patent Application 06996 (published 28 December 1983). The significance of the methanol-to-olefins (MTO) type processes, especially for producing ethene, is discussed in *Hydrocarbon Processing*, November 1982, pp. 117—120. It is generally known that the MTO process can be optimized to produce a major fraction of $C_2$—$C_4$ olefins; however, there is also produced a significant $C_5^+$ byproduct, including polymethylbenzenes, such as durene, as described in U.S. Patent 4,025,576 and various proposals have been made to recover ethene and other gases from byproduct water and $C_5^+$ hydrocarbon liquids. Treatment of the $C_5^+$ liquids to dealkylate the polymethylbenzenes has been necessary to convert this fraction into a satisfactory liquid fuel, for instance as described in U.S. Patents 4,347,397 and 4,387,261. However, such processes add significantly to the cost of the liquid fuels plant.

The present invention is based on the observation that methanol and/or DME may be converted into liquid fuels, particularly distillate, in a multistage continuous process, with integration between the major process units. The initial stage MTO type process hydrocarbon effluent stream, after byproduct water separation, is fed directly to the MOGD section without prior fractionation or treatment of heavier hydrocarbons. In particular, it has been found that durene-containing $C_5^+$ hydrocarbons can be fed to the oligomerization unit with lower olefins. Heavy aromatics are passed through with the MOGD distillate product, which may be post-hydrogenated to saturate the olefinic components and hydrogenate aromatics. The hydrotreated distillate product thus obtained has a high cetane number and excellent properties for use as diesel fuel or jet fuel.

According to the present invention, there is provided a continuous process for converting an oxygenated organic feedstock into liquid hydrocarbons comprising the steps of

(a) contacting the feedstock with at least one primary stage dehydration catalyst comprising ZSM-5 typs zeolite at an elevated temperature and a moderate pressure to convert at least a portion of the feedstock into hydrocarbons containing a major fraction of $C_2$—$C_4$ olefins and a minor fraction containing $C_5^+$ heavy hydrocarbons;

(b) cooling and separating the dehydration effluent from step (a) to provide an aqueous liquid stream, a heavy hydrocarbon liquid stream and a light hydrocarbon vapor stream rich in $C_2$—$C_4$ olefins;

(c) pressurizing and heating at least a portion of the olefinic light hydrocarbon stream and substantially all of the heavy hydrocarbon liquid stream from step (b) to form a secondary stage olefinic feedstream;

(d) contacting the olefinic feedstream from step (c) in a secondary stage with an oligomerization catalyst comprising a medium-pore, shape-selective, acidic zeolite at substantially increased pressure and moderate temperature to convert olefins into a heavier liquid hydrocarbon effluent stream comprising olefinic gasoline and distillate range liquids;

(e) fractionating the liquid hydrocarbon effluent stream from step (d) to obtain a distillate stream, an olefinic gasoline stream and a ligher hydrocarbon stream;

(f) recycling at least a portion of the olefinic gasoline stream from step (e) to step (d); and

(g) hydrogenating olefinic distillate from step (e) to provide a stabilized distillate product.

The invention also provides apparatus for use in carrying out such a process comprising

(i) a first stage reactor for dehydration catalyst;

(ii) a separator operatively connected to an outlet of reactor (i) for separating water from the reactor effluent;

(iii) a second stage reactor for oligomerization catalyst operatively connected to an outlet for hydrocarbon effluent from separator (ii);

(iv) a fractionator operatively connected to an outlet for oligomerized product from reactor (iii) for fractionating oligomerized product into a light hydrocarbon fraction rich in $C_3$ and $C_4$ aliphatic hydrocarbons, a $C_5^+$ gasoline fraction and a distillate fraction;

(v) means operatively connected to an outlet for the gasoline fraction from the fractionator (iv) for recycling at least some of the gasoline fraction to the second stage reactor (iii); and

(vi) a third stage reactor for hydrotreating catalyst operatively connected to an outlet for distillate from the fractionator (iv) and having an outlet for stabilized distillate product.

Catalyst versatility permits the same zeolite catalyst to be used in both the primary dehydration stage (MTO) and the secondary oligomerization stage (MOGD) of the process of the invention. While it is also possible to employ substantially different catalysts in these stages, it is advantageous to employ a standard ZSM-5 having a silica alumina molar ratio of about 70:1 in both stages.

Preferred oligomerization catalysts for use in the process include the crystalline aluminosilicate zeolites having a silica to alumina ratio of at least 12, a constraint index of 1 to 12 and acid cracking activity of 160 to 200. Examples of such ZSM-5 type zeolites are ZSM-5, ZSM-11, ZSM-12, ZSM-22, ZSM-23, ZSM-35 and ZSM-38. ZSM-5 is described in U.S. Patents 3,702,886 and Re. 29,948; ZSM-11 is described in U.S. Patent 3,709,979; ZSM-12 is described in U.S. Patent 3,832,449; ZSM-23 is described in U.S. Patent 4,076,842; ZSM-35 is described in U.S. Patent 4,016,245; and ZSM-38 is described in U.S. Patent No. 4,046,839. An especially suitable catalyst for fixed bed operations is HZSM-5 zeolite composited with 35 weight % alumina binder in the form of 1 to 5 mm cylindrical extrudates.

These medium pore shape selective catalysts are sometimes known as porotectosilicates or "Pentasil" catalysts. Other catalysts suitable for converting methanol/DME into lower olefins are zeolite ZSM-45 and those described in U.S. Patents 4,393,265 and 4,387,263 and EP—A—81683 (published 22 June 1983). In addition to the preferred aluminosilicates, the borosilicate, ferrosilicate and "silicalite" materials may be employed. ZSM-5 type catalysts are particularly advantageous because the same material may be employed for dehydration of methanol into DME, conversion into lower olefins and oligomerization.

In the following description, the process of the invention is described with reference to a plurality of fixed catalyst bed reactors operated under differing process conditions depending upon relative position in the system. However, it should be understood that various other reactor configurations may be used, including fluidized bed catalytic reactors, moving bed reactors and fixed bed reactors.

The invention will now be described in greater detail by way of example only with reference to the accompanying drawings, in which:

Fig. 1 is a process flow sheet showing the major unit operations and process streams;

Fig. 2 is a schematic representation of a preferred multi-stage reactor system and fractionation system for ethane recovery;

Fig. 3 is a typical olefin conversion reactor system for distillate mode operation; and

Fig. 4 is a schematic representation of an alternative system.

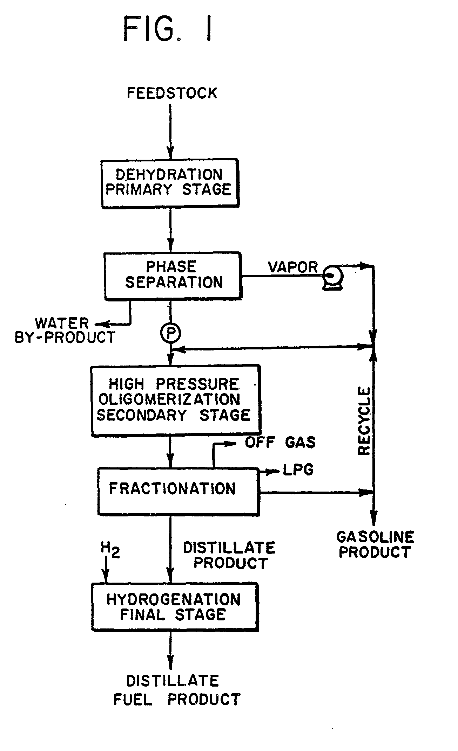

Referring to Fig. 1 of the drawings, process feedstock (methanol or DME) is fed to a primary stage where it is converted into lower olefins and gasoline hydrocarbons plus water by dehydration. Byproduct water is recovered by simple phase separation from the cooled effluent. Liquid hydrocarbons comprising $C_5^+$ gasoline range materials are pumped to a higher secondary stage pressure. This stream may contain as much as 6 to 10 weight % durene. At least a portion of the vapor phase effluent from the primary stage is compressed and heated along with the liquids to oligomerization reaction temperature, and the combined olefinic stream (optionally containing recycled olefinic gasoline) is reacted at high pressure and elevated temperature. The secondary stage effluent is then separated into light gases, $C_5^+$ gasoline for recycle in part and distillate range hydrocarbons. The distillate stream contains a major fraction of high boiling aliphatics and a minor amount of aromatics. Hydrotreating (HDT) in the final stage is a relatively mild process to saturate olefinic compounds and convert aromatics into corresponding naphthenes without substantial cracking or dealkylation to yield a distillate fuel product. Ethene may be recovered as a valuable chemical feedstock from the process.

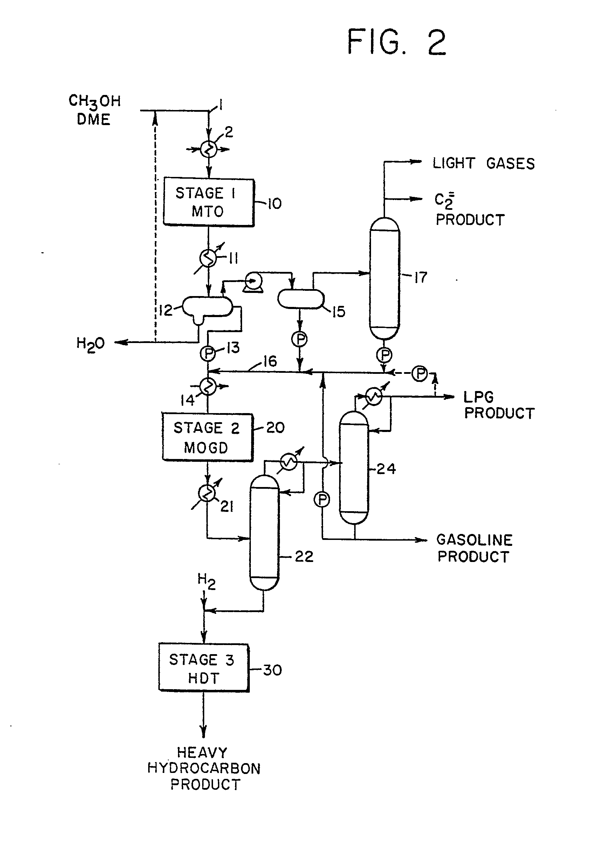

In the process for the conversion of olefins into heavier hydrocarbons by catalytic oligomerization using an acid crystalline zeolite, for example a ZSM-5 type zeolite catalyst, process conditions can be varied to favor the formation of either gasoline or distillate range products. At moderate temperatures and relatively high pressures, the conversion conditions favor a distillate range product having a normal boiling point of at least 165°C. Lower olefinic feedstocks containing $C_2$—$C_6$ alkenes may be converted selectively; however, the distillate mode conditions do not convert the major fraction of the ethene. While 50 to 95% of the propene, butene-1 and others may be converted in the distillate mode, only about 10 to 50% of the ethene component will be consumed. Accordingly, the ethene is advantageously recovered prior to the secondary oligomerization stage, as shown in Fig. 2, which depicts a preferred system for converting methanol and/or DME.

In the primary stage, ethene production may be optimized by employing fixed bed conditions including a temperature of about 260°C to 425°C, a pressure of about 170 to 800 kPa and weight hourly space velocity of about 0.5 to 1.0 based on ZSM-5 equivalent catalyst and methanol equivalent in the feedstock. Typically about 25 to 90% of MeOH/DME feedstock is converted per pass and water diluent is included in the feedstock in a water to methanol and/or dimethyl ether molar ratio of about 0.1:1 to 5:1. Under these conditions, the primary stage hydrocarbon effluent usually contains about 25 to 40 weight % ethene, about 10 to 50 weight % propene, about 2 to 30 weight % butene, less than 10 weight % $C_1$ to $C_4$ paraffins, and about 5 to 20 weight % aromatics, including about 1 to 5 weight % durene.

Referring now to Fig. 2, the feedstock is methanol, which may be partially dehydrated in a separate process step over a gamma-alumina catalyst to yield dimethyl ether (DME) and water. A preliminary dewatering step can be used to provide a feedstock consisting essentially of $CH_3OH$ and/or DME; however, the presence of water in the MTO reactor may be beneficial. The feedstock is fed continuously under low pressure through line 1 and heat exchanger 2 where it is raised to process temperature and introduced into the first stage MTO reactor system 10. The first stage effluent is cooled in heat exchanger 11 to condense water and a major amount of $C_5^+$ hydrocarbons. These liquids are separated from the hydrocarbon vapor in phase separator 12. Byproduct water may be recovered from unreacted feedstock and discarded or a portion may be recycled.

The liquid hydrocarbon phase from separator 12 is then brought up to distillate mode oligomerization pressure by a pump 13, heated in heat exchanger 14 and introduced into the secondary stage reactor system 20. The ethene-rich hydrocarbon vapor stream from separator 12 is compressed and condensed liquid, mainly $C_3$—$C_4$ olefins, is separated in high pressure separator 15, after which it is pumped to secondary stage pressure and combined through conduit 16 with other hydrocarbon liquids. Ethene product and other $C_2^-$ light gases are recovered from the vapor stream by fractionator unit 17, which may be a cryogenic still, for example. Methane and ethane offgas may be removed from the system at this point.

Pressurized $C_3^+$ hydrocarbons from fractionator 17 are combined with other hydrocarbon liquid streams through conduit 16 and passed to the second stage MOGD reactor system 20 under high pressure at moderate temperature. As discussed below, the preferred MOGD reactor system is a multizone arrangement. The second stage effluent is cooled in heat exchanger 21 and fractionated in distillation tower 22, from which the distillate range liquids boiling above about 165 to 175°C are fed as a continuous stream to the third stage HDT reactor 30. Gasoline, rich in $C_5^+$ olefins and lighter hydrocarbons are further fractionated in tower 24 to provide an olefinic gasoline stream for recycle to the MOGD reactor system or recovery as product. The lighter hydrocarbons, rich in $C_3$—$C_4$ alkenes may be condensed and recovered as LPG product or optionally recycled to the MOGD reactor system.

The main concept is to cascade substantially all $C_3^+$ hydrocarbon first stage product into an MOGD reactor followed by hydrotreating of the distillate product. This minimizes the number of process steps and maximizes distillate production by polymerizing gasoline range olefins, and by alkylating gasoline range aromatics. Durene will be reduced via saturation to its corresponding naphthene in the hydrotreating step.

A typical MTO operation is conducted over a fixed bed of HZSM-5/alumina extrudate catalyst at about 170 kPa with a 1:1 $H_2O:CH_3OH$ equivalent ratio at 315°C and a space velocity (WHSV) of 0.5 to 1 to convert about 50% of the oxygenated organic feedstock components into hydrocarbons. The following Table lists the organic product distribution from a typical MTO run.

TABLE
MTO Product Distribution

| Component | wt.% |
|---|---|
| Methane | 0.6 |
| Ethene | 26.2 |
| Ethane | 0.1 |
| Propene | 22.8 |
| Propane | 3.9 |
| Butenes | 7.9 |
| Isobutane | 3.9 |
| n-Butane | 2.6 |
| Pentenes | 2.4 |
| $C_5$ P+N | 7.1 |
| $C_6$ P+N | 5.1 |
| $C_6O$ | 0.6 |
| $C_7$ P+N | 3.2 |
| $C_7O$ | 0.7 |
| $C_8$ P+O+N | 2.1 |
| $C_9$ P+O+N | 1.3 |
| $C_{10}$ P+O+N | 1.1 |
| Benzene | 0.1 |
| Toluene | 0.5 |
| $C_8$ Aromatics | 3.5 |
| $C_9$ Aromatics | 2.1 |
| $C_{10}$ Aromatics | 2.2 |
| (Durene | 1.7) |

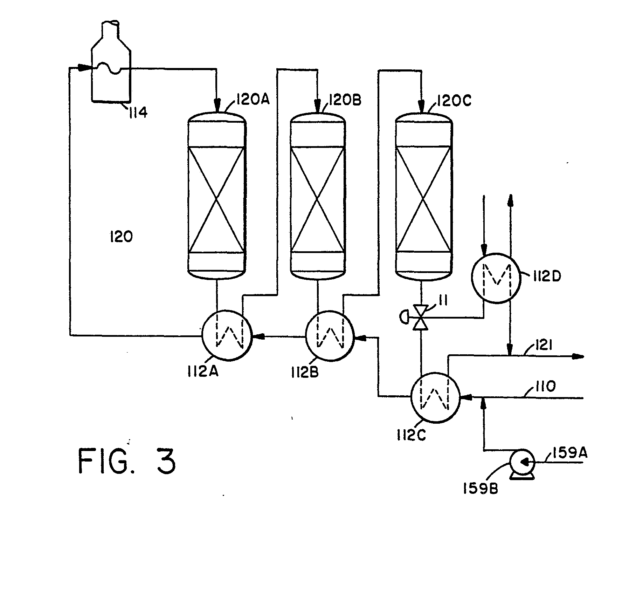

A typical distillate mode secondary stage reactor system 120 is shown in Fig. 3. A plural reactor system may be employed with inter-reactor cooling, whereby the reaction exotherm can be carefully controlled to prevent excessive temperature above the normal moderate range of about 190° to 315°. The olefinic feedstream is introduced through conduit 110 and carried by a series of conduits through heat exchangers 112A, B, C and furnace 114 where the feedstream is heated to reaction temperature. The olefinic feedstock is then carried sequentially through a series of reactors 120A, B, C containing zeolite catalyst beds in which at least a portion of the olefin content is converted into heavier distillate constituents. Advantageously, the maximum temperature differential across any one reactor is about 30°C and the space velocity (LHSV based on olefin feed) is about 0.5 to 1.5. The heat exchangers 112A and 112B provide inter-reactor cooling and 112C cools the effluent to separation temperature. An optional heat exchanger 112D may recover further heat from the effluent stream 121 prior to separation. Gasoline from recycle conduit 159A is pressurized by pump means 159B and combined with the feedstream, preferably at a ratio of about 1 to 3 parts by weight per part of olefin in the secondary stage feedstream.

Preferably, the secondary stage process conditions are optimized to produce heavy liquid hydrocarbons having a normal boiling point greater than about 175°C, and employs a fixed bed of ZSM-5 type catalyst to oligomerize olefins at a start of cycle temperature of about 230 to 260°C and pressure of about 4200 to 7000 kPa.

Rather than recover ethene and other light gases from the first stage effluent, ethene can be sent to the MOGD reactor through which it will pass relatively unreacted, thus reducing the amount of $C_3^+$ in the ethylene separation plant and improving its efficiency.

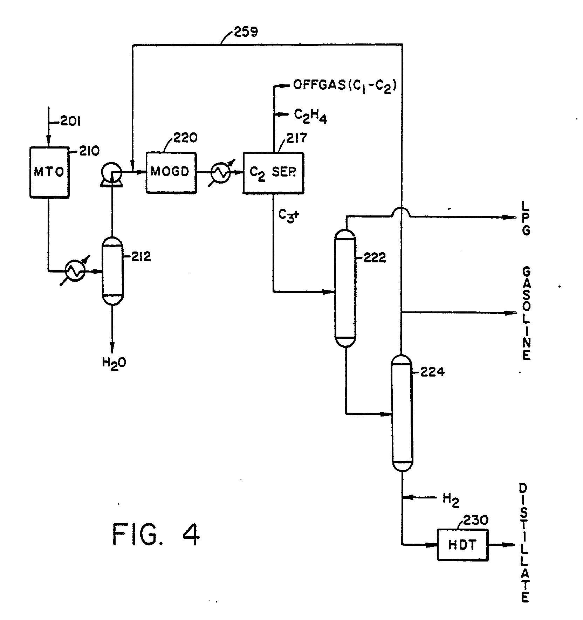

An alternative system depicted schematically in Fig. 4, provides for passing oxygenate feedstock 201 through the primary stage 210 and water separation unit 212 and cascading substantially the entire organic effluent to the secondary stage 220, operating in maximum distillate mode. Unreacted ethene and other light gases (for example, methane and ethane) are recovered in deethanizer unit 217 and the $C_3^+$ hydrocarbons are fractionated first in a debutanizer unit 222 and then splitter tower 224. Olefinic gasoline is recovered as product and partially recycled through line 259 to the MOGD reactor 220. The distillate range bottoms stream is hydrogenated to stabilize the olefinic components and reduce polyalkylbenzenes to naphthenes. This results in distillate yield being increased by polymerization of the $C_5^+$ olefins (which normally go to gasoline), and by aromatic/olefin alkylation in the MOGD reactor. Gasoline end point is also lowered to send the durene to the product hydrotreater 230. The durene (along with other heavy aromatics) is then saturated to tetramethylcyclohexane, thus eliminating the need for a dealkylation unit. Aromatics' saturation also increases the cetane value of the distillate product.

The hydrogenation step may employ a hydrogenation catalyst comprising Group VIIIA and Group VIA (IUPAC) metals under mild hydrotreating conditions to convert durene into a naphthene compound and to saturate olefinic components. Preferred catalyst comprises Co or Ni together with W or Mo, and/or noble metals, such as Pt and Pd. A suitable HDT process is described in U.S. Patent 4,211,640. Similar hydrogenation processed adapted for continuous operation are known in petroleum refining technology.

While the primary stage dehydration reactor has been exemplified by a fixed bed unit, a suitable fluid catalyst apparatus is described in U.S. Patent 4,379,123.

Compared to a conventional system in which MTO liquids containing gasoline range materials and durene are recovered and severaly hydrotreated to reduce the durene content by dealkylation, the present system is an economic process for increasing the relative amount of distillate. Typically, an increase of 40% or more of high quality fuel can be achieved. The amount of aromatics in the gasoline is also decreased from about 22% to 15%. The preferred distillate mode operation can provide a larger part of the total fuel products as heavy hydrocarbons, usually in a distillate/gasoline ratio of about 1.3 to 6:1, while retaining a high yield of valuable ethylene product. Substantially all of the polymethylbenzenes or other aromatics formed in the dehydration reactor stage are accumulated in the distillate fraction, and the hydrotreated distillate durene content is decreased substantially below 2 weight %, preferably below 1 weight %.

The process of the invention is particularly useful for producing a major product stream in which the 175°C$^+$ fraction consists mainly of $C_{10}$ to $C_{20}$ aliphatic hydrocarbons containing a minor amount of cyclic components. The low temperature, high pressure distillate mode secondary stage operation favors the formation of linear aligomers.

By integration of dehydration, oligomerization and hydrotreating, a route is provided for converting MTO products into distillate with a minimum of processing steps. This technique reduces capital cost and provides an economic process for the production of distillate fuels and ethene, with gasoline and LPG being made in minor amount.

**Claims**

1. A continuous process for converting an oxygenated organic feedstock into liquid hydrocarbons comprising the steps of

(a) contacting the feedstock with at least one primary stage dehydration catalyst comprising ZSM-5 type zeolite at an elevated temperature and a moderate pressure to convert at least a portion of the feedstock into hydrocarbons containing a major fraction of $C_2$—$C_4$ olefins and a minor fraction containing $C_5^+$ heavy hydrocarbons;

(b) cooling and separating the dehydration effluent from step (a) to provide an aqueous liquid stream, a heavy hydrocarbon liquid stream and a light hydrocarbon vapor stream rich in $C_2$—$C_4$ olefins;

(c) pressurizing and heating at least a portion of the olefinic light hydrocarbon stream and substantially all of the heavy hydrocarbon liquid stream from step (b) to form a secondary stage olefinic feedstream;

(d) contacting the olefinic feedstream from step (c) in a secondary stage with an oligomerization catalyst comprising a medium-pore, shape-selective, acidic zeolite at substantially increased pressure and moderate temperature to convert olefins into a heavier liquid hydrocarbon effluent stream comprising olefinic gasoline and distillate range liquids;

(e) fractionating the liquid hydrocarbon effluent stream from step (d) to obtain a distillate stream, an olefinic gasoline stream and a lighter hydrocarbon stream;

(f) recycling at least a portion of the olefinic gasoline stream from step (e) to step (d); and

(g) hydrogenating olefinic distillate from step (e) to provide a stabilized distillate product.

2. A process according to claim 1, wherein the primary dehydration stage feedstock comprises methanol and/or dimethyl ether and is converted over a HZSM-5 type zeolite catalyst into hydrocarbons comprising a major amount of $C_2$—$C_4$ olefins and a minor amount of normally liquid hydrocarbons containing durene.

3. A process according to claim 2, wherein the primary dehydration stage is carried out a temperature of 260 to 425°C, a pressure of 170 to 800 kPa and a weight hourly space velocity of 0.5 to 1.0 based on ZSM-5 equivalent catalyst and methanol equivalent in the primary stage feedstock, over a fixed bed catalyst.

4. A process according to claim 3, wherein the feedstock includes water in an amount such that the water to methanol and/or dimethyl ether molar ratio is 0.1:1 to 5:1.

5. A process according to any one of claims 1 to 4, wherein the primary stage effluent is cooled in step (b) under process pressure and light hydrocarbon vapors are compressed in step (c) to form a second liquid hydrocarbon stream which is fed under process pressure to the secondary stage, step (d).

6. A process according to claim 5, wherein the compressed light hydrocarbon vapor is fractionated to recover an ethene-rich product stream.

7. A process according to any one of claims 1 to 6, wherein the secondary stage employs a fixed bed of ZSM-5 type zeolite catalyst and olefins are oligomerized at a temperature of 190 to 315°C and pressure of 4200 to 7000 kPa.

8. Apparatus for use in the process according to claim 1, comprising

(i) a first stage reactor for dehydration catalyst;

(ii) a separator operatively connected to an outlet of reactor (i) for separating water from the reactor effluent;

(iii) a second stage reactor for oligomerization catalyst operatively connected to an outlet for hydrocarbon effluent from separator (ii);

(iv) a fractionator operatively connected to an outlet for oligomerized product from reactor (iii) for fractionating oligomerized product into a light hydrocarbon fraction rich in $C_3$ and $C_4$ aliphatic hydrocarbons, a $C_5^+$ gasoline fraction and a distillate fraction;

(v) means operatively connected to an outlet for the gasoline fraction from the fractionator (iv) for recycling at least some of the gasoline fraction to the second stage reactor (iii); and

(vi) a third stage reactor for hydrotreating catalyst operatively connected to an outlet for distillate from the fractionator (iv) and having an outlet for stabilized distillate product.

**Patentansprüche**

1. Kontinuierliches Verfahren zur Umwandlung eines Sauerstoff-enthaltenden organischen Ausgangsmaterials in flüssige Kohlenwasserstoffe, welches die Schritte umfaßt:

(a) Kontakt dieses Ausgangsmaterials mit zumindest einem Vorstufen-Dehydrierungskatalysator, der einen Zeolith vom Typ ZSM-5 umfaßt, bei erhöhter Temperatur und mäßigem Druck, um zumindest einen Teil dieses Ausgangsmaterials in Kohlenwasserstoffe umzuwandeln, die eine Hauptfraktion von $C_2$—$C_4$-Olefinen und eine geringe Fraktion enthalten, die schwere $C_5^+$-Kohlenwasserstoffe enthält;

(b) Abkühlung und Trennung des Dehydrierungsabflusses aus dem Schritt (a), um einen wässrigen flüssigen Strom, einen flüssigen Strom schwerer Kohlenwasserstoffe und einen Dampfstrom leichter Kohlenwasserstoffe zu schaffen, der reich an $C_2$—$C_4$-Olefinen ist;

c) Komprimieren und Erwärmen zumindest eines Teils des olefinischen Stroms leichter Kohlenwasserstoffe und im wesentlichen des gesamten flüssigen Stromes schwerer Kohlenwasserstoffe aus dem Schritt (b), um einen olefinischen Zufuhrstrom für die Sekundärstufe zu bilden;

(d) Kontakt des olefinischen Zufuhrstroms aus dem Schritt (c) in der Sekundärstufe mit einem Oligomerisierungskatalysator, der einen formselektiven, sauren Zeolith mit mittleren Poren umfaßt, bei wesentlich erhöhtem Druck und mäßiger Temperatur, um Olefine in einen Abflußstrom schwererer, flüssiger Kohlenwasserstoffe umzuwandeln, der olefinisches Benzin und Flüssigkeiten im Destillatbereich umfaßt;

(e) Fraktionieren des Abflußstromes flüssiger Kohlenwasserstoffe aus dem Schritt (d), um einen Destillatstrom, einen olefinischen Benzinstrom und einen Strom leichterer Kohlenwasserstoffe zu erhalten;

(f) Kreislaufführung zumindest eines Teils dieses olefinischen Benzinstroms vom Schritt (e) zum Schritt (d) und

(g) Hydrierung des olefinischen Destillats vom Schritt (e), um ein stabilisiertes Destillatprodukt zu schaffen.

2. Verfahren nach Anspruch 1, worin das Zufuhrmaterial zur Hydrierungsvorstufe Methanol und/oder Dimethylether umfaßt, und über einem Zeolith vom Typ HZSM-5 in Kohlenwasserstoffe umgewandelt wird, die eine Hauptmenge an $C_2$—$C_4$-Okefinen und eine geringe Menge Durol enthaltender normaler flüssiger Kohlenwasserstoffe umfassen.

3. Verfahren nach Anspruch 2, worin die Dehydrierungsvorstufe bei einer Temperatur von 260 bis 425°C, einem Druck von 170 bis 800 kPa und einer stündlichen Gewichts-Raum-Geschwindigkeit von 0,5 bis 1,0, bezogen auf die äquivalente Menge des Katalysators ZSM-5 und die äquivalente Methanolmenge im Ausgangsmaterial für die Vorstufe, über einem Festbettkatalysator durchgeführt wird.

4. Verfahren nach Anspruch 3, worin das Ausgangsmaterial Wasser in einer solchen Menge umfaßt, daß das Molverhältnis von Wasser zu Methanol und/oder Dimethylether 0,1:1 bis 5:1 beträgt.

5. Verfahren nach einem der Ansprüche 1 bis 4, worin der Abfluß der Vorstufe im Schritt (b) bei Verfahrensdruck abgekühlt wird und die leichten Kohlenwasserstoffdämpfe im Schritt (c) komprimiert werden, um einen zweiten flüssigen Kohlenwasserstoffstrom zu bilden, der unter Verfahrensdruck zur Sekundärstufe Schritt (d) zugeführt wird.

6. Verfahren nach Anspruch 5, worin der komprimierte Dampf der leichten Kohlenwasserstoffe fraktioniert wird, um einen an Ethan reichen Produktstrom zu gewinnen.

7. Verfahren nach einem der Ansprüche 1 bis 6, worin die Sekundärstufe ein Festbett eines Zeolithkatalysators vom Typ ZSM-5 umfaßt und die Olefine bei einer Temperatur von 190 bis 315°C und einem Druck von 4200 bis 7000 kPa oligomerisiert werden.

8. Vorrichtung zur Verwendung um Verfahren nach Anspruch 1, welche umfaßt:

(i) einen Vorstufenreaktor für den Dehydrierungskatalysator,

(ii) einen Separator, der wirksam an den Auslaß des Reaktors (i) verbunden ist, um Wasser vom Reaktorabfluß abzutrennen,

(iii) einen Sekundärstufenreaktor für den Oligomerisierungskatalysator, der wirksam an den Auslaß für den Kohlenwasserstoffabfluß aus dem Separator (ii) verbunden ist.

(iv) einen Fraktionator, der wirksam an den Auslaß für das oligomerisierte Produkt aus dem Reaktor (iii) verbunden ist, um das oligomerisierte Produkt in eine leichte Kohlenwasserstofffraktion, die reich an aliphatischen $C_3$— und $C_4$-Kohlenwasserstoffen ist, eine $C_5^+$-Benzinfraktion und eine Destillatfraktion zu fraktionieren,

(v) eine Einrichtung zur Kreislaufführung von zumindest einem Teil der Benzinfraktion zum Sekundärstufenreaktor (iii), die wirksam an den Auslaß für die Benzinfraktion aus dem Fraktionator (iv) verbunden ist, und

(vi) einen Reaktor der dritten Stufe zur Hydrobehandlung des Katalysators, der wirksam an den Auslaß für das Destillat aus dem Fraktionator (iv) verbunden ist und einen Auslaß für das stabilisierte Destillatprodukt aufweist.

**Revendications**

1. Un procédé de conversion en continu d'une charge organique oxygénée en hydrocarbures liquides comprenant les étapes suivantes:

a) dans un étage primaire on met la charge au contact d'au moins un catalyseur de déshydratation comprenant une zéolite de type ZSM-5, à température élevée et sous pression modérée pour convertir qu'au moins une partie de la charge soit convertie en hydrocarbures contenant une fraction majeure d'oléfines en $C_2$ à $C_4$ et une fraction mineure enfermant des hydrocarbures lourds en $C_5^+$;

b) on refroidit et on sépare l'effluent de déshydratation provenant de l'étape (a) pour former un courant aqueux liquide, un courant liquide d'hydrocarbures lourds et un courant de vapeur d'hydrocarbures légers, riche en oléfines en $C_2$ à $C_4$;

c) on met sous pression et on chauffe au moins une partie du courant d'hydrocarbures oléfiniques légers et sensiblement tout le courant liquid d'hydrocarbures lourds provenant de l'étape (b) pour former un courant de charge oléfinique pour un étage secondaire;

d) dans un étage secondaire, on met le courant de charge oléfinique provenant de l'étape (c) au contact d'un catalyseur d'oligomérisation comprenant une zéolite acide sélective de forme à pores moyens, à pression sensiblement accrue et à température modérée, pour convertir les oléfines en un courant effluent formé d'hydrocarbures liquides plus lourds comprenant une essence oléfinique et des liquides bouillants dans l'intervalle des distillats;

e) on fractionne le courant effluent d'hydrocarbures liquides provenant de l'étape (d) pour obtenir un courant de distillat, un courant d'essence oléfinique et un courant d'hydrocarbures plus légers;

f) on recycle au moins une partie du courant d'essence oléfinique provenant de l'étape (e) vers l'étape (d); et

g) on hydrogène le distillat oléfinique provenant de l'étape (e) pour obtenir un distillat stabilisé.

2. Un procédé selon la revendication 1, dans lequel la charge de l'étage primaire de déshydratation comprend du méthanol et/ou du diméthyléther et on la convertit sur un catalyseur zéolitique de type HZSM-5 de hydrocarbures comprenant une quantité majeure d'oléfines en $C_2$ à $C_4$ et une quantité mineure d'hydrocarbures normalement liquides contenant du durène.

3. Un procédé selon la revendication 2, dans lequel l'étape de déshydratation primaire est mise en oeuvre à une température de 260 à 425°C, une pression de 170 à 800 kPa et une vitesse spatiale horaire pondérale de 0,5 à 1,0 sur la base du catalyseur équivalent à la ZSM-5 et à un équivalent méthanol dans la charge de l'étape primaire, sur un catalyseur en lit fixe.

4. Un procédé selon la revendication 3, dans lequel la charge comprend de l'eau en quantité telle que le rapport molaire eau/méthanol et/ou diméthyléther est compris entre 0,1/1 et 5/1.

5. Un procédé selon l'une quelconque des revendications 1 à 4, dans lequel l'effluent de l'étape primaire est refroidi dans l'étape (b) à la pression du procédé et les vapeurs d'hydrocarbures légers sont comprimées dans l'étape (c) pour former un deuxième courant d'hydrocarbures liquides qui est introduit à la pression du procédé dans l'étape secondaire, l'étape (d).

6. Un procédé selon la revendication 5, dans lequel la vapeur d'hydrocarbures légers comprimés est fractionnée pour récupérer un courant de produits riches en éthylène.

7. Un procédé selon l'une quelconque des revendications 1 à 6, dans lequel dans l'étape secondaire, on emploie un lit fixe de catalyseur zéolitique de type ZSM-5 et les oléfines sont oligomérisées à une température de 190 à 315°C et sous une pression de 4200 à 7000 kPa.

8. Dispositif utilisable dans le procédé selon la revendication 1, comprenant:

i) un premier étage de réacteur pour le catalyseur de déshydratation;

ii) un séparateur relié opérationnellement à une sortie du réacteur (i) pour séparer l'eau de l'effluent du réacteur;

iii) un deuxième étage de réacteur pour le catalyseur d'oligomérisation relié opérationnellement à une sortie de l'effluent d'hydrocarbures provenant du séparateur (ii);

(iv) un dispositif de fractionnement relié opérationnellement à une sortie du produit oligomérisé provenant du réacteur (iii) destiné au fractionnement du produit oligomérisé en une fraction d'hydrocarbures légers riche en hydrocarbures aliphatiques en $C_3$ et en $C_4$, une fraction d'essence en $C_5+$ et une fraction de distillat;

v) des moyens reliés opérationnellement à une sortie de la fraction d'essence provenant de l'installation de fractionnement (iv) pour recycler au moins une partie de la fraction d'essence vers le second étage de réacteur (iii);

vi) un troisième étage de réacteur destiné à l'hydrotraitement du catalyseur relié opérationnellement à une sortie du distillat provenant de l'installation de fractionnement (iv) et comportant une sortie pour le distillat stabilisé.

# FIG. 1

FEEDSTOCK

DEHYDRATION
PRIMARY STAGE

PHASE
SEPARATION → VAPOR

WATER
BY-PRODUCT

HIGH PRESSURE
OLIGOMERIZATION
SECONDARY STAGE

→ OFF GAS

FRACTIONATION → LPG

RECYCLE

H₂      DISTILLATE
PRODUCT

GASOLINE
PRODUCT

HYDROGENATION
FINAL STAGE

DISTILLATE
FUEL PRODUCT

1

# FIG. 2

FIG. 3

FIG. 4